(19) Europäisches Patentamt / European Patent Office / Office européen des brevets

(11) **EP 4 018 928 A1**

(12) **EUROPEAN PATENT APPLICATION**

(43) Date of publication:
**29.06.2022 Bulletin 2022/26**

(21) Application number: **20306678.2**

(22) Date of filing: **23.12.2020**

(51) International Patent Classification (IPC):
*A61B 5/11* (2006.01)    *A61B 5/00* (2006.01)
*A43B 3/00* (2022.01)    *A43B 17/00* (2006.01)

(52) Cooperative Patent Classification (CPC):
**A43B 17/00; A43B 3/38; A61B 5/112;**
**A61B 5/1121; A61B 5/6829**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Feetme**
**75019 Paris (FR)**

(72) Inventor: **JACOBS, Damien**
**75019 Paris (FR)**

(74) Representative: **Icosa**
**83 avenue Denfert-Rochereau**
**75014 Paris (FR)**

(54) **FOOTWEAR AND METHOD FOR FOOT VELOCITY ESTIMATION**

(57)    The present invention relates to a footwear comprising an inertial measurement unit (IMU); a pressure sensor (PS); and a data processing unit configured to estimate gait data by applying a particle filter.

**FIG.1**

## Description

### FIELD OF INVENTION

The present invention pertains to the field of systems, methods, and wearable devices for gait analysis. In particular, the invention relates to footwear associated with a data processing unit for obtaining and/or estimating gait data.

### BACKGROUND OF INVENTION

Accurate estimation of foot velocity is desirable in several applications in rehabilitation, in assessment of energy expenditure, in sport, as well as to advance research on gait disorders.

Previous works have attempted to estimate foot velocity by using footwear-mounted sensors, such as inertial measuring units.

Inertial measurement units can be used to estimate the foot velocity via the accelerometry technique, in which foot velocity is estimated by integration of the acceleration recorded by the accelerometer of the inertial measurement unit. This acceleration integration has to take into account the orientation calculated by the integration of the angular velocity recorded by the gyroscope of the inertial measurement unit.

However, this technique requires a correction step for estimating and subtracting the gravitational component from the acceleration data sensed by the accelerometer. Moreover, in order to obtain the velocity, the corrected acceleration data are integrated using initial conditions which are uncertain, thereby requiring to make assumptions about the initial conditions. Also, the drifts from the inertial measurement unit can bias the calculation of the velocity integration and they have to be corrected to get an accurate estimation of the velocity or the spatial trajectory.

With most accelerometry techniques, velocity can be accurately estimated only if an assumption of a consistent style of walking or running is made, and they lose in accuracy if the subject wearing a foot-mounted inertial measuring unit alternates walking and running phases, or if he/she moves over a varied terrain, thereby causing non-predictable variations of the point of contact with the ground.

Assumptions such as those mentioned hereabove introduce a strong limitation and make the gait data estimation methods difficult to generalize. Moreover, these methods cannot be applied to gait data estimation in presence of gait disorders, since most of the commonly adopted assumptions, such as initial conditions, style of walking or running, or point of contact with the ground, do not apply therein.

Therefore, there is a need for an improved footwear and footwear-mounted sensors for accurate gait data estimation, especially for velocity estimation.

It is an objective of the present invention to provide footwear and methods that overcome current limitations and allow to accurately determine foot velocity.

It is also an objective of the present invention to provide footwear and methods for determining foot velocities in real-life conditions, which is not limited to the laboratory setting, and which can be easily generalized.

### SUMMARY

The present invention relates to a footwear comprising:

- an inertial measurement unit;
- a pressure sensor; and
- a data processing unit obtaining and estimating gait data based on measurements of the inertial measurement unit and the pressure sensor, the data processing unit being configured to estimate the gait data by applying a particle filter;

wherein the inputs of the update step of the particle filter comprise the current IMU velocity determined during a stance; and
wherein the current IMU velocity is estimated with the angular velocity of the IMU around the center of pressure and the distance between the IMU and the center of pressure during a stance.

In one embodiment, the inputs of the update step of the particle filter are asynchronous with a predetermined gait stage.

In one embodiment, the current IMU velocity is estimated by the following equation:

$$\vec{v} = R(\vec{\omega} \wedge \vec{r}) \,(2)$$

in which $\vec{\omega}$ is the angular velocity of the IMU, $\vec{r}$ is the vector joining the IMU and the center of pressure during a stance, $\wedge$ stands for cross product and R is the rotation matrix between the fixed reference frame and the IMU rotational frame.

In one embodiment, the pressure sensor PS comprises a plurality of pressure cells, preferably capacitive pressure cells.

The plurality of pressure cells may be comprised in an insole.

In one embodiment, the footwear of the present invention further comprises a computing unit configured to determine the position of the center of pressure based on the measurements of the plurality of pressure cells.

In one embodiment, the inertial measurement unit comprises at least one accelerometer and/or at least one gyroscope.

In one embodiment, the particle filter is a Kalman filter.

The present invention further relates to a method of processing gait data in a footwear implemented by a data processing unit configured to process the gait data with a particle filter recursively repeating a prediction step and an update step, the method comprising:

- obtaining pressure data from a pressure sensor and determining the position of center of pressure during a stance;
- obtaining the angular velocity of an inertial measurement unit bonded to the footwear;
- estimating current IMU velocity with the angular velocity of the IMU around the center of pressure and the distance between the IMU and the center of pressure during a stance; and
- introducing the current IMU velocity in the particle filter as a first input during the following update step.

In one embodiment, the method of the present invention further comprises:

- receiving and introducing prior measurement and gait data in the particle filter during the preceding prediction step, so as to obtain a predicted IMU velocity;
- introducing the predicted IMU velocity in the particle filter as a second input during the following update step, so as to estimate an updated IMU velocity.

In one embodiment, the steps of the present method are repeated during a stance, preferably at least 5 times during a stance. In particular, the steps of the method are repeated at a repetition frequency corresponding to at least one of: the sampling frequency of the angular velocity $\vec{\omega}$ by the IMU and the sampling frequency of PS.

In an embodiment, the particle filter is a Kalman filter.

The present invention further relates to a computer program product comprising instructions which, when the program is executed by a computer, cause the computer to carry out the steps of the method as described hereabove.

### DEFINITIONS

In the present invention, the following terms have the following meanings:

- **"stance"** refers to the interval of time during which the footwear is in contact with the ground.

- **"swing",** refers to the interval of time during which the footwear is not in contact with the ground.

- **"center of pressure",** refers to the weighted barycenter of an area where pressure is applied. In particular, during stance, the center of pressure is the point where the equivalent force of weight is applied by the foot to the footwear.

- **"particle filter"** refers to a computer-implemented algorithm which recursively repeats a prediction step and an update or correction step, in order to estimate the state of a dynamic system when the measurements of the system state contain uncertainties or errors.

- **"Kalman filter"** refers to a particle filter in which the uncertainties or errors of the measured values are approximated with a Gaussian distribution.

- **"prior measurement"** refers to the gait measurement introduced as an input in the prediction step of a particle filter.

- **"prediction step"** refers to the step of a particle filter which generates gait data on the basis of prior measurements and gait data, wherein the $n^{th}$ prediction step generates gait data at a time to on the basis of prior measurements and gait data obtained at a time $t=t_0-\Delta t$ preceding to and/or generated in the $(n-1)^{th}$ update step of the particle filter; and wherein the gait data generated in the $n^{th}$ prediction step are further introduced as an input in the $n^{th}$ update step of the particle filter.

- **"predicted state"** refers to the gait data, along with their uncertainties, generated in the prediction step of a particle filter.

- **"observational measurements"** refer to the new observational measurements, along with their noises, that are inputs of the update step.

- **"update step"** refers to the step of a particle filter which generates gait data, along with their uncertainties, on the basis of the predicted state and of new observational measurements, such as the foot velocity, wherein the $n^{th}$ update step generates gait data at a time to on the basis of:

    o the predicted state generated in the $n^{th}$ prediction step, and
    o the observations obtained from measurements performed at a time to;

and wherein the gait data generated in the $n^{th}$ update step are further introduced as an input in the $(n+1)^{th}$ prediction step of the particle filter.

- **"updated state"** refers to gait data, along with their errors, generated in the update step of a particle filter, such as for instance the gait data, along with their errors, obtained in the update step of a Kalman filter.

- **"data processing unit"** is herein not restricted to hardware capable of executing software, and refers in a general way to a processing device, which can for example include a microprocessor, an integrated circuit, a field-programmable gate array (FPGA) or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a RAM (Random-Access Memory) or a ROM (Read-Only Memory). Instructions may be notably stored in hardware, software, firmware or in any combination thereof.

## DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

This invention relates to a footwear comprising an inertial measurement unit IMU; a pressure sensor PS; and a data processing unit obtaining and estimating gait data based on measurements of the inertial measurement unit IMU and the pressure sensor PS.

### The footwear: IMU

Inertial measurement units IMU comprise at least one accelerometer for measuring linear accelerations and/or at least one gyroscope for measuring angular velocities.

Based on the measurements of the inertial measurement unit IMU, it is possible to obtain linear velocities and positions, for instance by integrating linear accelerations over time. However, the linear velocities and positions thus obtained will drift over time from the real linear velocity and real position, due to the accumulation of errors. Therefore, they need to be periodically corrected.

Examples of devices comprising an inertial measurement unit IMU are: QuantiMotion (Bonsai Systems, Zurich, Switzerland), MetaMotionR (MbientLab, San Francisco, CA, USA), NilsPod (Portabiles, Erlangen, Germany), Move 4 (movisens, Karlsruhe, Germany), PhysilogR 5 (Gait Up, Lausanne, Switzerland), EXL-s3 (EXEL, Bologna, Italy) and Shimmer 3 (Shimmer Research, Dublin, Ireland).

In one embodiment of the present invention, the inertial measurement unit IMU comprises at least one accelerometer, configured to measure the linear acceleration $\vec{a}$ along at least one axis, with respect to the rotational frame of the IMU.

In one embodiment, the accelerometer of the inertial measurement unit IMU is a three-axis accelerometer that measures the components of the linear acceleration $\vec{a}$ along three orthogonal axes ($x, y, z$) of the IMU rotational frame.

In one embodiment, the accelerometer of the inertial measurement unit IMU has a measurement range comprised between $\pm$ 2g and $\pm$ 16g; preferably, the accelerometer measurement range is equal to $\pm$ 8g.

In one embodiment, the accelerometer of the inertial measurement unit IMU has a sampling frequency comprised between 10 Hz and 10000 Hz, preferably between 100 Hz and 500 Hz.

In one embodiment of the present invention, the inertial measurement unit IMU comprises at least one gyroscope. The gyroscope of the inertial measurement unit IMU is configured to measure an angular velocity $\vec{\omega}$ about at least one axis, with respect to the IMU rotational frame.

In one embodiment, the gyroscope of the inertial measurement unit IMU has a measurement range comprised between $\pm$125 deg/s and $\pm$2000 deg/s; preferably, the gyroscope measurement range is equal to $\pm$500 deg/s.

In one embodiment, the gyroscope of the inertial measurement unit IMU has a sampling frequency comprised between 10 Hz and 2000 Hz, preferably between 100 Hz and 500 Hz.

Advantageously, gyroscopes are insensitive to the influence of gravity. Moreover, measurements of gyroscopes are not biased due to movement artifacts during the impact of the heel with the ground at the beginning of a stance, *i.e.* when deceleration is large. To the contrary, accelerometers are less adapted when deceleration speed is coupled with high deceleration intensity.

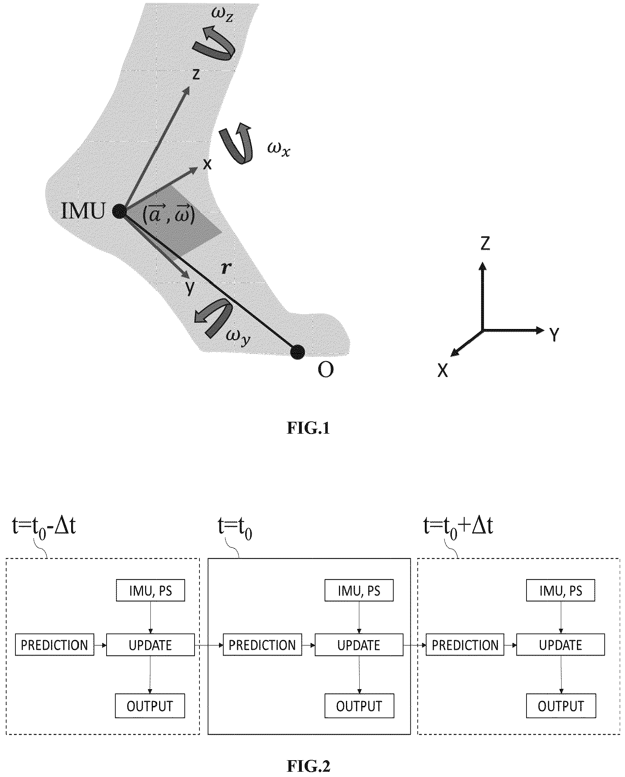

In one embodiment, the gyroscope of the inertial measurement unit IMU is a three-axis gyroscope that measures the components $\omega_x$, $\omega_y$, $\omega_z$ of the angular velocity co along three orthogonal axes (x, y, z) of the IMU rotational frame. One example of this embodiment is represented in figure 1.

In a preferred embodiment, the inertial measurement unit IMU comprises at least one gyroscope and at least one accelerometer.

In one embodiment, the inertial measurement unit IMU comprises a magnetometer. The magnetometer can be included in the IMU or coupled to the IMU.

### The footwear: pressure sensor

The footwear according to the present invention comprises a pressure sensor PS. The pressure sensor provides with a measure of intensity of pressure and the location of the center of pressure, *i.e.* the point where the equivalent force of weight is applied by the foot to the footwear.

In one embodiment, the pressure sensor PS has a sampling frequency comprised between 50 Hz and 250 Hz, preferably between 90 Hz and 150 Hz.

In one embodiment, the pressure sensor PS has a sampling frequency equal to 100 Hz.

In one embodiment, the footwear comprises a plurality of pressure cells.

In one embodiment, the plurality of pressure cells is comprised in an insole adapted to be included in the footwear.

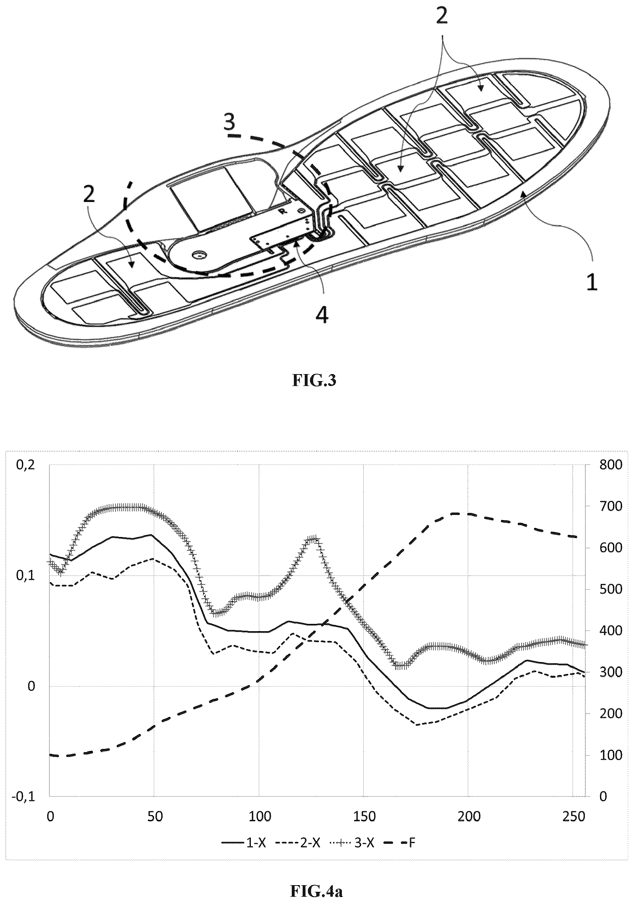

One example of this embodiment is illustrated in figure 3. In this example, the pressure sensor comprises 18 pressure cells 2 distributed over a measuring area 1. The central part 3 of the insole (delimited by a dotted line open circle) comprises IMU and data processing unit. A bus 4 allows for electrical connection between pressure cells and data processing unit via wires.

In a preferred embodiment, the plurality of pressure cells are resistive pressure cells or capacitive pressure cells.

Resistive pressure cells may be of any type, in particular sensors comprising two layers of flexible film covered with a thin layer of conductive material and a resistive ink, such as the FlexiForce™ Sensors (Tekscan, Inc., MA, USA).

Capacitive pressure cells may be of any type, in particular sensors comprising a thin sheet of deformable dielectric material placed between two electrodes.

Indeed, the value of the capacitance C of a capacitive pressure cell can be determined as a function of the thickness L of the dielectric sheet of the capacitive pressure cells, the surface S of the upper electrode and the lower electrode of the capacitive pressure cells and the dielectric constant $\varepsilon$ of the material between the electrodes by the following equation:

$$C = \frac{\varepsilon S}{L}$$

Consequently, when the thickness L of the dielectric sheet of the capacitive pressure cells is changed, the capacitance C varies. Change of thickness may be induced by a force or a pressure, for instance by application of a mass over the cell, as it is the case during gait.

According to one embodiment, the footwear according to the present invention further comprises a computing unit configured to determine the position of the center of pressure (O) based on the measurements of the plurality of capacitive pressure sensors.

### The footwear: data processing unit

In one embodiment, the data processing unit is configured to estimate the gait data by applying a particle filter.

The data processing unit of the present invention may be a microprocessor, an integrated circuit, a field-programmable gate array (FPGA) or a programmable logic device (PLD).

For instance, the data processing unit may be a microcontroller unit (MCU). Examples of MCUs comprise the Cortex-Mx, -Rx (Arm, Cambridge, UK) MCUs such as the STMicroelectronics STM32L4 series MCUs.

In the present invention, the data processing unit is configured to implement a particle filter, in particular a Kalman filter.

### The particle filter

Particle filters, or Sequential Monte Carlo (SMC) methods, are a family of computer-implemented algorithms used in signal processing to estimate the state of a dynamic system. The term "particles" refers to the possible states in which a dynamic system can be found.

The true value of the state of a dynamic system is not accessible. Partial information about the true value can be obtained via observations, such as sensor measurements, which are affected by statistical noise.

In this context, particle filters allow to obtain, by recursively repeating prediction steps and update steps (also referred to as correction step) the best estimate of the state of the dynamic system, *i.e.* an estimate which is close to the true value of the dynamic system state.

Each prediction step of a particle filter receives a prior measurement and it generates a predicted state.

Each update step of a particle filter receives the predicted state as well as observational measurements with noise, and it generates an updated state which is closer to the true state of the dynamic system, when compared to the measurements.

In the present invention, the prediction step of the particle filter receives, as inputs, a prior measurement and the gait data generated in the previous update step of the particle filter and/or initial values. On the basis of both information, the prediction step of the particle filter generates a predicted state, *i.e.,* a prediction of the next state of the system. Said predicted state is then provided as input in the following update step of the particle filter.

The update step combines the predicted state and the observational measurements, which comprises gait data calculated or estimated on the basis of the measurements of the inertial measuring unit IMU and the pressure sensor PS.

Especially, each update step of the particle filter receives the observational measurement and combines it with the predicted state. The update step then generates an updated state based on the combination of the observational measurement and the predicted state, by means of a weighted gain. Since such combination is affected by independent noise, from the measurement, or by the uncertainty from the state prediction, their weighting in the update step allows to obtain an updated state which is closer to the true state.

In some implementations of the particle filter, the possible states of the system are determined and, in the update step, a weight is associated to each possible state. The weight is selected based on the combination of the measurements and the predicted state. These possible states are often referred to as "particles". Such weight represents the probability of a "particle", *i.e.* of a possible state, of being close to the true value of the state of the dynamic system.

The updated state obtained from the update step of a particle filter has a twofold function: to more accurately estimate the true state of the system on the basis of the measurements, and to predict the next state.

Indeed, the update state is, in turn, introduced as an input in the following prediction step, and is used to generate the following predicted state.

The reintroduction of the update step in the following prediction step is repeated recursively over time.

Examples of particle filters include, but are not limited to: particle Markov chain Monte Carlo algorithms, regularized particle filters, sampling importance resampling (SIR) filters, auxiliary sampling importance resampling (ASIR) filters, Kalman filters.

One example of implementation of the particle filter of the present invention is illustrated in figure 2. In this particular embodiment, the particle filter is a Kalman filter.

Advantageously, the particle filter of the present invention allows to reduce the errors of gait data obtained in the prediction step, by combining them with the measurements of the inertial measuring unit IMU and the pressure sensor PS. The updated state obtained from such combination is closer to the true state of the system, when compared to the gait data obtained on the basis of the inertial measuring unit IMU alone. Therefore, the model of the movement of the footwear therefrom obtained is more accurate.

In the present invention, by state of the dynamic system it is meant gait data, along with their errors.

In a preferred embodiment, the gait data are the IMU velocities, positions and/or orientations.

### The Kalman filter

In one embodiment, the particle filter of the present invention is a Kalman filter.

The Kalman filter is a type of particle filter in which the statistical noise of the measured values is approximated with a Gaussian distribution. Therefore, it can be parameterized by means and covariances.

Kalman filters are commonly used in inertial navigation systems. In this context, Kalman filters are used to combine the data measured by the inertial navigation systems to the estimations of the state of a moving object, such as for instance the estimation of the position of a vehicle or the velocity of an aircraft. The Kalman filter takes into account the errors of the acceleration and angular velocity from the inertial navigation system as parameters for acceleration and angular velocity and provides an updated state which more accurately describes the true state, such as the position or velocity, of the dynamic system.

Kalman filters are particle filters that allow to obtain, by recursively repeating a prediction steps and an update steps, an estimate of the true state of the dynamic system, while reducing at the same time the computational load.

One example of implementation of the prediction step and the update step of a particle filter in the present invention is described hereunder. In this particular example, the particle filter is a Kalman filter.

The $n^{th}$ prediction step of a Kalman filter executed at a time to produces a predicted state on the basis of: an update state which is obtained at a time $t=t_0-\Delta t$ preceding to, and the observational measurements.

The $n^{th}$ predicted state requires prior measurements and gait data obtained before implementation of the $n^{th}$ prediction step, for instance it may comprise constants and/or gait data generated in the $(n-1)^{th}$ update step of the Kalman filter. Subsequently, the predicted state is provided as input in the $n^{th}$ update step of the Kalman filter. The $n^{th}$ update step of the Kalman filter further receives a new observational measurement such as the foot velocity estimate, which is combined to the predicted state, so as to generate the updated state. Such updated state, in turn, is provided as an input to the $(n+1)^{th}$ prediction step.

In figure 2, the $(n-1)^{th}$ prediction step, the $(n-1)^{th}$ update step, the $(n+1)^{th}$ prediction step and the $(n+1)^{th}$ update step of the Kalman filter are represented in the dotted line. The $n^{th}$ prediction step and the $n^{th}$ update step are represented in the continuous line. The initial values are not illustrated.

### The Kalman filter - prediction step

In the embodiment illustrated in figure 2, the prior gait state received in the $n^{th}$ prediction step comprise the data of the updated state generated in the $(n-1)^{th}$ update step of the Kalman filter. The gait data and its related errors, *i.e.* the covariance matrix, of the prediction state are calculated on the basis of the measurements of the inertial measurement unit IMU at the time t= to and the previous updated state at the time $t= t_0-\Delta t$. Contrarily to the predicted state, the gait data and its related errors of the updated state are calculated or estimated on the basis of the measurements of the inertial measurement unit IMU and the pressure sensor PS at the time $t=t_0-\Delta t$ preceding to.

### The Kalman filter - update step

As described hereabove, the update step of the Kalman filter generates an updated gait state on the basis of the predicted gait state and on the basis of observational measurements, such as the measurements from the inertial measurement unit IMU and the pressure sensor PS.

In one embodiment, the observational measurement comprises the current IMU velocity obtained from the measurements of the inertial measurement unit IMU and the pressure sensor PS. In this embodiment, the update step of the Kalman filter receives the current IMU velocity determined during a stance, and estimates an updated IMU velocity. The update step combines the predicted state and the observational measurements, for estimating the gain correction. This gain is applied to the predicted state to provide a more accurate estimation of the real IMU velocity.

In one embodiment, the current IMU velocity is calculated by equation (1)

$$\vec{v} = R\left(\vec{\omega} \wedge \vec{r} + \frac{d\vec{r}}{dt}\right) (1)$$

wherein $\vec{\omega}$ is the angular velocity of the inertial measurement unit IMU, $\vec{r}$ is the vector joining the inertial measurement unit IMU and the center of pressure O of the pressure sensor PS, $\wedge$ stands for cross product and R is the rotation matrix between the fixed reference frame and the IMU rotational frame.

In some instances, the term $d\vec{r}/dt$ of equation (1) is negligible. Indeed, during a stance and roll of the foot, the principal movement of the stance foot is rotation, and its translation is negligible.

Especially, during a stance the stance foot passes from a heel strike position to a toe-off position. In the heel strike position, the stance foot executes a first rotation about the stance heel, then, in the toe-off position, the stance foot executes a second rotation about the stance toe.

Meanwhile, the contralateral foot is in a swing phase of the gait cycle, and it advances in front of the body.

Since the main movement of the stance foot is a rotation, and its translation is negligible, the term $d\vec{r}/dt$ is in general inferior to 0.01m/s during a stance, hence negligible.

In this case, the current IMU velocity may be estimated by equation (2)

$$\vec{v} = R\left(\vec{\omega} \wedge \vec{r}\right) (2).$$

In equation (2) the current IMU velocity in the fixed reference frame (X, Y, Z) is estimated as the moment between the angular velocity $\vec{\omega}$ and the position vector joining the inertial measurement unit IMU and the center of pressure O in the IMU rotational frame (x, y, z).

Advantageously, the estimation of the IMU velocity on the basis of the angular velocity $\vec{\omega}$ and the position vector $\vec{r}$ does not require any assumption. Therefore, the present IMU velocity calculation or estimation is robust and can be generalized to a wide number of applications without additional assumptions.

In one embodiment, the inertial measurement unit IMU comprises at least one gyroscope configured to measure the angular velocity $\vec{\omega}$ in the fixed reference frame of the ground. In a preferred embodiment, the angular velocity $\vec{\omega}$ is estimated in the second half of the stance, *i.e.* between mid-stance and terminal stance, when the pressure exerted on pressure sensor is high (the second foot is in swing, thus all weight is exerted on one foot). Advantageously, the angular velocity $\vec{\omega}$, and hence the IMU velocity, obtained with this embodiment are less affected by movement artifacts.

The inertial measurement unit IMU is comprised within the footwear, hence its reference frame is attached to the same reference frame of the foot of a subject wearing said footwear and it is in movement with respect to the fixed reference frame of the ground.

Therefore, in the present invention the coordinates of the position vector joining the center of pressure O and the measurement unit IMU are transformed into the coordinate system of the fixed reference frame of the ground by the rotation matrix R, in order to obtain the IMU velocity.

In figure 1, the axes of the rotational frame of the inertial measurement unit IMU are (x, y, z) and the axes of the fixed reference frame of the ground are (X, Y, Z).

In one embodiment of the present invention, the current IMU velocity is introduced as an input in the update step of the particle filter.

Subsequently, the update step of the particle filter generates an updated IMU velocity based on the predicted IMU velocity and the current IMU velocity.

Advantageously, the updated IMU velocity is closer to the real velocity of the footwear when compared to the predicted IMU velocity, since the particle filter has removed sources of high uncertainty due to cumulated drifts from IMU, which are associated with the measurements of the inertial measurement unit IMU over long periods of time.

As aforementioned, the stance phase is the interval of time during which the footwear is in contact with the ground. Several gait events occur during a stance, such as for instance: the stance heel strike, the contralateral toe off, the full forefoot load, the stance heel rise.

These gait events define the beginning and the end of the stance sub-phases. Indeed, the stance phase is divided in sub-phases, such as:

- the loading response phase, which may last 5% to 20% of the whole stance duration;
- the stance foot single support phase, which usually lasts 70% to 80% of the whole stance duration;
- the pre-swing phase, which may last 5% to 20% of the whole stance duration.

In one embodiment of the present invention, the inputs of the update step of the particle filter are asynchronous with a predetermined gait event. In other words, it is not required that input of the update step of the particle filter be precisely associated with one event of stance, such as the instant when foot velocity is null for instance - identified by change of sign of acceleration.

In this embodiment, the IMU velocity may be estimated or calculated at any instant of time during a stance, be it an instant of time corresponding to a predetermined gait event, or an instant of time in between two consecutive gait events.

The computational load is thus reduced, because the detection of gait events is not needed to estimate or calculate the IMU velocity.

Moreover, in this embodiment the IMU velocity estimation or calculation is not restricted to predefined gait events occurring during a stance, but it can be performed at any instant of time, thereby allowing to obtain a large number of IMU velocities during a stance.

Accordingly, a large number of prediction/update cycles of the particle filter can be implemented during a stance.

For instance, in this embodiment more than 5 prediction/update cycles are implemented during a stance, preferably more than 10.

**The method**

The present invention also relates to a method of processing gait data in a footwear with a data processing unit configured to process said gait data by means of a particle filter recursively repeating a prediction step and an update step.

The method according to the present invention comprises a step of obtaining pressure data from a pressure sensor PS and determining the position of the center of pressure O during a stance, in the reference frame of the inertial measurement unit IMU.

The method further comprises a step of obtaining the angular velocity of an inertial measurement unit (IMU) bonded to the footwear.

The method further comprises a step of estimating current IMU velocity with the angular velocity of the IMU around the center of pressure and the distance between the IMU and the center of pressure during a stance. This is typically done by transformation of the cross product of angular velocity with the vector joining the IMU and the center of pressure from the rotational frame of the inertial measurement unit IMU to the fixed reference frame of the ground.

Finally, the method further comprises a step of introducing the current IMU velocity in the particle filter as a first input during the following update step.

In one embodiment, the method comprises two additional steps. A first step of receiving and introducing prior measurement and gait data in the particle filter during the preceding prediction step, so as to obtain a predicted IMU velocity and a second step of introducing the predicted IMU velocity in the particle filter as a second input during the following update step, so as to output an updated IMU velocity.

In one embodiment, the current IMU velocity is estimated by equation (2)

$$\vec{v} = R(\vec{\omega} \wedge \vec{r})\,(2)$$

in which $\vec{\omega}$ is the angular velocity of the IMU, $\vec{r}$ is the vector joining the IMU and the center of pressure O during a stance, $\wedge$ stands for cross product and R is the rotation matrix between the fixed reference frame and the IMU rotational frame.

In one embodiment, the step of obtaining pressure data from a pressure sensor PS and determining the position of the center of pressure O during a stance comprises:

- determining a gait phase of the footwear based on the data from a pressure sensor PS;
- if the footwear is in a stance phase, determining the position of the center of pressure O,

wherein the steps of determining the gait phase of the footwear and determining the position of the center of pressure O described hereabove are repeated at a predefined repetition frequency. This embodiment allows to make a difference between stance phase and swing phase.

In one embodiment, the steps of the method are repeated during a stance, preferably at least 5 times during a stance. In particular, the steps of the methods may be repeated at a repetition frequency which is a function of at least one of the followings: the sampling frequency of velocity of IMU; the sampling frequency of the accelerometer of the inertial measurement unit IMU; the sampling frequency of the gyroscope of the inertial measurement unit IMU and the sampling frequency of the pressure sensor PS. With a plurality of predictions and updates during one stance, the accuracy of foot velocity estimation is improved during stance, which gives also a better estimate of foot velocity during swing phase.

In one embodiment, said repetition frequency is comprised between 50 Hz and 250 Hz. In a preferred embodiment, the repetition frequency is comprised between 90 Hz and 150 Hz.

In one embodiment, the step of determining a gait phase of the footwear comprises: comparing the data from the pressure sensor PS with a predefined pressure threshold; and, if the data from the pressure sensor PS are above said predefined pressure threshold, determining the gait phase of the footwear as a stance phase; whereas if the data from the pressure sensor PS are inferior or equal to said predefined pressure threshold, determining the gait phase of the footwear as a swing phase.

In one embodiment, the pressure sensor PS comprises a plurality of pressure cells adapted to be included in the footwear, and the data from the pressure sensor PS is the set of the pressures sensed by each pressure cell of the plurality of pressure cells.

In one embodiment, said particle filter is a Kalman filter and the current IMU velocity is introduced, as input, in the update step of the Kalman filter.

In this embodiment, the observational measurement of IMU velocity is further introduced, as input, in the update step of the Kalman filter. By IMU velocity it is meant the IMU velocity at a time to which is measured after the prediction step of the Kalman filter, on the basis of the measurements of the inertial measurement unit IMU and the pressure sensor PS at a time $t=t_0-\Delta t$ preceding to.

The present invention also relates to a computer program comprising instructions of processing which, when the program is executed by a computer, cause the computer to carry out the steps of the method described above.

While various embodiments have been described and illustrated, the detailed description is not to be construed as being limited hereto. Various modifications can be made to the embodiments by those skilled in the art without departing from the true spirit and scope of the disclosure as defined by the claims.

**BRIEF DESCRIPTION OF THE DRAWINGS**

Figure 1 is a schema of a foot, the reference frame of the foot being attached to the reference frame of the footwear of the present invention (not illustrated) according to a particular embodiment in which the inertial measurement unit IMU comprises a 3-axis gyroscope for measuring the angular velocity $\vec{\omega}$ on the basis of the angular velocities ωx, coy, coz along three orthogonal axes (x,y,z) in the IMU rotational frame.

Figure 2 is a flowchart representing a particular embodiment of the present invention, in which the particle filter implemented by the data processing unit is a Kalman filter.

Figure 3 is a perspective view of a pressure sensor in the form of an insole, suitable to be inserted into an article of footwear.

Figures 4a-4c are graphs showing the x-, y- and z-component of foot velocity, measured during one stance.

**EXAMPLES**

The present invention is further illustrated by the following example.

3 participants wearing the footwear of the present invention took part in the study. Signals were collected from a commercial IMU (LSM6DS33, STMicro-Electronics) comprising a ±8 g accelerometer and ±500deg/s gyroscope at a sampling frequency of 150 Hz.

In parallel, true velocity was measured using video recording and image analysis (motion capture system). This method is known to be very accurate.

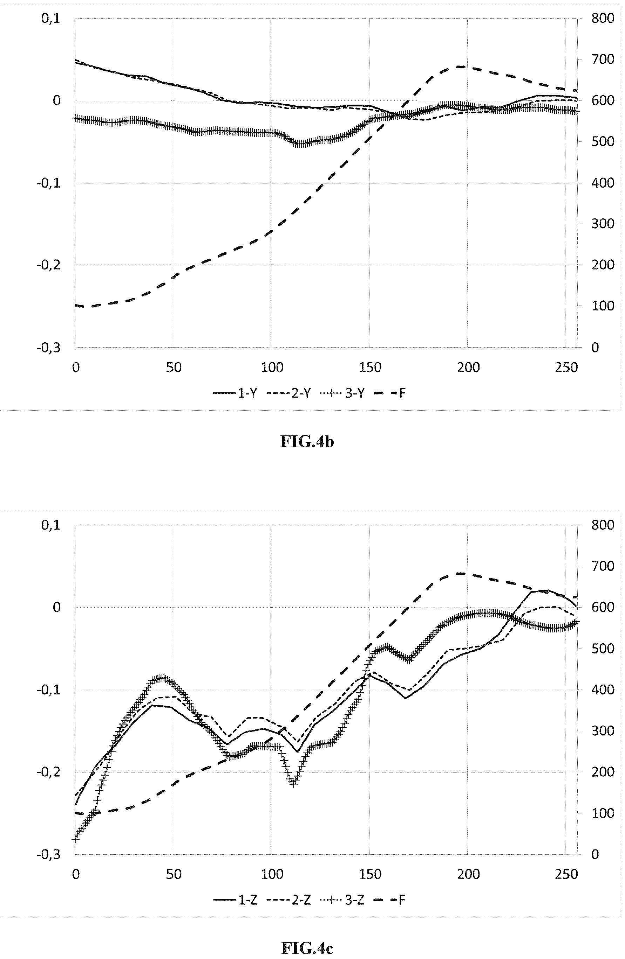

Figures 4a to 4c show the foot velocity components (in x, y and z directions, left axis in m.s⁻¹) versus time (in ms) obtained during a stance:

- by video recording and image analysis (solid line, reference: 1-X, 1-Y or 1-Z)
- with the present footwear and running a Kalman filter with 15 updates during stance. (dashed line, reference: 2-X, 2-Y or 2-Z)
- with a prior art device including solely an IMU and running a Kalman filter method in which one update is done during stance when foot velocity is identified to be zero ("hairy" line, reference: 3-X, 3-Y or 3-Z).

In addition, figures 4a to 4c show the total force exerted on the pressure sensor (bold dashed line, reference: F, arbitrary unit). This curve enables to identify the various gait events.

These results show that foot velocity estimated according to the invention is very accurate, with low deviation from true foot velocity. However, this accuracy is obtained with a simple device included in the footwear, allowing for continuous measurements and without the complex environment of video recording and computing means of image analysis.

Besides, comparing with prior art device using solely an IMU device, accuracy is improved in a range of 5% to 25%.

**Claims**

1. A footwear comprising

   • an inertial measurement unit (IMU);
   • a pressure sensor (PS); and
   • a data processing unit obtaining and estimating gait data based on measurements of the inertial measurement unit (IMU) and the pressure sensor PS, the data processing unit being configured to estimate the gait data by applying a particle filter;

   wherein the inputs of the update step of the particle filter comprise the current IMU velocity determined during a stance; and
   wherein the current IMU velocity is estimated with the angular velocity of the IMU around the center of pressure (O) and the distance between the IMU and the center of pressure (O) during a stance.

2. The footwear according to claim 1, wherein the inputs of the update step of the particle filter are asynchronous with a predetermined gait stage.

3. The footwear according to claim **1** or **2**, wherein the current IMU velocity is estimated by equation (2)

$$\vec{v} = R(\vec{\omega} \wedge \vec{r}) \ (2)$$

in which $\vec{\omega}$ is the angular velocity of the IMU, $\vec{r}$ is the vector joining the IMU and the center of pressure (O) during a stance, $\wedge$ stands for cross product and R is the rotation matrix between the fixed reference frame and the IMU rotational frame.

4. The footwear according to any one of claims **1** to **3**, wherein the pressure sensor PS comprises a plurality of pressure cells, preferably capacitive pressure cells.

5. The footwear according to claim **4**, wherein the plurality of pressure cells is comprised in an insole.

6. The footwear according to claim **4** or **5**, wherein the footwear further comprises a computing unit configured to determine the position of the center of pressure (O) based on the measurements of the plurality of pressure cells.

7. The footwear according to any one of claims **1** to **6**, wherein the IMU comprises at least one accelerometer and/or at least one gyroscope.

8. The footwear according to any one of claims **1** to **7**, wherein the particle filter is a Kalman filter.

9. A method of processing gait data in a footwear implemented by a data processing unit configured to process the gait data with a particle filter recursively repeating a prediction step and an update step, the method comprising:

- obtaining pressure data from a pressure sensor (PS) and determining the position of center of pressure (O) during a stance;
- obtaining the angular velocity of an inertial measurement unit (IMU) bonded to the footwear;
- estimating current IMU velocity with the angular velocity of the IMU around the center of pressure (O) and the distance between the IMU and the center of pressure (O) during a stance; and
- introducing the current IMU velocity in the particle filter as a first input during the following update step.

10. The method of processing gait data according to claim **9**, further comprising:

- receiving and introducing prior measurement and gait data in the particle filter during the preceding prediction step, so as to obtain a predicted IMU velocity;
- introducing the predicted IMU velocity in the particle filter as a second input during the following update step, so as to estimate an updated IMU velocity.

11. The method of processing gait data according to any one of claims **9** to **10**, wherein the current IMU velocity is estimated by equation (2)

$$\vec{v} = R(\vec{\omega} \wedge \vec{r}) \ (2)$$

in which $\vec{\omega}$ is the angular velocity of the IMU, $\vec{r}$ is the vector joining the IMU and the center of pressure (O) during a stance, $\wedge$ stands for cross product and R is the rotation matrix between the fixed reference frame and the IMU rotational frame.

12. The method of processing gait data according to any one of claims **9** to **11**, wherein the steps of the method are repeated during a stance, preferably at least 5 times during a stance.

13. The method of processing gait data according to claim **12**, wherein the steps of the method are repeated at a repetition frequency corresponding to at least one of: the sampling frequency of the angular velocity $\vec{\omega}$ of IMU and the sampling frequency of PS.

14. The method of processing gait data according to any one of claims **9** to **13**, wherein the particle filter is a Kalman filter.

15. A computer program product comprising instructions which, when the program is executed by a computer, cause the computer to carry out the steps of the method according to any one of claims **9** to **14**.

**FIG.1**

**FIG.2**

**FIG.3**

**FIG.4a**

FIG.4b

FIG.4c

Europäisches
Patentamt

European
Patent Office

Office européen
des brevets

## EUROPEAN SEARCH REPORT

Application Number

EP 20 30 6678

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/318972 A1 (COUVET SERGE [FR]) 8 October 2020 (2020-10-08) * paragraph [0052] - paragraph [0108]; claim 1; figures 1-10 * | 1-15 | INV. A61B5/11 A61B5/00 A43B3/00 A43B17/00 |
| X | US 2018/303634 A1 (CASLER RICK [US] ET AL) 25 October 2018 (2018-10-25) * paragraph [0015] - paragraph [0368]; claims 70,82; figures 1-19 * | 1-15 | |
| X | US 2017/055880 A1 (AGRAWAL SUNIL K [US] ET AL) 2 March 2017 (2017-03-02) * paragraph [0052] - paragraph [0108]; claim 1; figures 1-10 * | 1 | |
| Y | WO 2020/216816 A1 (CENTRE NAT RECH SCIENT [FR]; UNIV PARIS [FR] ET AL.) 29 October 2020 (2020-10-29) * page 7, line 21 - page 39 * | 1-15 | |
| Y | US 2013/338961 A1 (YOUSSEF JOE [FR] ET AL) 19 December 2013 (2013-12-19) * paragraph [0049] - paragraph [0249]; claims 16,26; figures 1-6 * | 1-15 | TECHNICAL FIELDS SEARCHED (IPC) A61B G01C A61F A43B |

The present search report has been drawn up for all claims

| Place of search | Date of completion of the search | Examiner |
|---|---|---|
| The Hague | 17 June 2021 | Munteh, Louis |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

......................................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 30 6678

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2021

| Patent document cited in search report | | Publication date | Patent family member(s) | | Publication date |
|---|---|---|---|---|---|
| US 2020318972 | A1 | 08-10-2020 | CA | 3025257 A1 | 30-11-2017 |
| | | | EP | 3465635 A1 | 10-04-2019 |
| | | | FR | 3051952 A1 | 01-12-2017 |
| | | | US | 2020318972 A1 | 08-10-2020 |
| | | | WO | 2017202948 A1 | 30-11-2017 |
| US 2018303634 | A1 | 25-10-2018 | US | 2010114329 A1 | 06-05-2010 |
| | | | US | 2018303634 A1 | 25-10-2018 |
| US 2017055880 | A1 | 02-03-2017 | US | 2017055880 A1 | 02-03-2017 |
| | | | WO | 2015164456 A2 | 29-10-2015 |
| WO 2020216816 | A1 | 29-10-2020 | EP | 3731238 A1 | 28-10-2020 |
| | | | WO | 2020216816 A1 | 29-10-2020 |
| US 2013338961 | A1 | 19-12-2013 | CN | 103314274 A | 18-09-2013 |
| | | | EP | 2646775 A1 | 09-10-2013 |
| | | | JP | 6015664 B2 | 26-10-2016 |
| | | | JP | 2014506318 A | 13-03-2014 |
| | | | KR | 20130127991 A | 25-11-2013 |
| | | | US | 2013338961 A1 | 19-12-2013 |
| | | | WO | 2012072957 A1 | 07-06-2012 |

EPO FORM P0459

For more details about this annex : see Official Journal of the European Patent Office, No. 12/82